# EUROPEAN PATENT APPLICATION

(11) **EP 4 599 768 A1**
(43) Date of publication of application: **13.08.2025**
(21) Application number: 24211609.3
(22) Date of filing: 08.11.2024
(51) Int. Cl.: A61B 5/339, A61B 5/349

(54) **LOCAL ECG ANNOTATION VISUALIZATION**

(30) Priority: 07.02.2024 US 202418435005
(71) Applicant: Biosense Webster (Israel) Ltd., 2066717 Yokneam (IL)
(72) Inventor: YARNITSKY, Jonathan, 2066717 Yokneam (IL); ALON, Talia, 2066717 Yokneam (IL); GREENBAUM, Lior, 2066717 Yokneam (IL)
(74) Representative: Carpmaels & Ransford LLP

(57) **Abstract**

Methods and systems for electrophysiological measurement involve obtaining signals that indicate cardiac electrical activity by using electrodes in contact with the body tissues of a living patient. These signals are then processed to identify local activation times at the electrodes. The identified local activation times are displayed as traces representing the signals over time on a display screen. The brightness of specific trace segments within a designated time window, encompassing the identified local activation times, may be increased relative to segments outside the time window. The method provides flexibility in signal acquisition, allowing for the use of intracardiac electrogram signals acquired within the heart or electrocardiogram signals acquired from the body surface. This method enhances visibility and enables efficient analysis of cardiac activity traces for diagnostic and monitoring purposes.

## Description

### CROSS-REFERENCE TO RELATED APPLICATIONS

This application claims the benefit of U.S. Provisional Patent Application 63/448,415, filed February 27, 2023, whose disclosure is incorporated herein by reference.

### FIELD

The present disclosure relates to electrophysiological measurements, and specifically to displaying electrophysiological signals.

### BACKGROUND

Electrophysiological measurements from human tissue may provide important diagnostic information about the state of the tissue. For example, signals indicative of cardiac electrical activity in a human heart are commonly recorded by measurement electrodes and analyzed for the purpose of measuring the local activation time (LAT) of the heart tissue at each electrode. The signal traces can be presented visually to a clinician as electrograms. An annotation point, representative of the LAT, can be marked automatically by superimposing an icon on the visual presentation of the respective electrogram. The clinician may utilize the LATs, as along with other characteristics of the electrograms, in assessing the state of the heart tissue.

The present disclosure will be more fully understood from the following detailed description of the examples thereof, taken together with the drawings in which:

### SUMMARY

According to disclosed embodiments of the invention, methods, apparatuses, and computer-readable media for electrophysiological measurement include acquiring signals indicative of cardiac electrical activity from electrodes in contact with tissues of a living patient's body. These acquired signals are then processed to identify local activation times at one or more of the electrodes. Another aspect may involve displaying traces representing the signals on a display screen as a function of time. In some aspects brightness of segments of the traces within a specified duration time window that contains the identified local activation times may increaserelative to parts of the traces outside the time window.

Additional aspects of the method include acquiring the signals by receiving intracardiac electrogram signals acquired within the patient's heart or electrocardiogram signals acquired from the patient's body surface. In certain aspects, the specified duration for the time window ranges from 10 ms to 100 ms. Furthermore, in one aspect systems and methods may ensure that the displayed traces on the screen are presented without overlaying icons that represent the local activation times.

### BRIEF DESCRIPTION OF THE DRAWINGS

Fig. 1 shows an example catheter-based electrophysiology mapping and ablation system, in accordance with an example of the disclosure;
Fig. 2 is a plot that schematically illustrates the identification of an annotation point in an electrophysiological signal, in accordance with an example of the disclosure; and
Fig. 3 is a schematic representation of a display including highlighted segments of recorded electrograms, in accordance with an example of the disclosure.

### DETAILED DESCRIPTION

### OVERVIEW

Measurements of local activation time (LAT) in cardiac tissue are useful, for example, in estimating the electrical conduction velocity (CV) in cardiac tissue, as well as in identifying arrhythmias and other pathologies. The local activation times can be identified automatically, using digital signal processing techniques, and may be indicated graphically by marking annotation points on the electrogram signal traces. Various algorithms may be used to choose the annotation point in a unipolar, bipolar, or multipolar electrogram signal. For example, the annotation point may indicate the point in time at which the timederivative of the signal reaches its lowest negative value.

In many systems, the signals received by the electrodes are presented on a display screen in the form of multiple parallel traces as a function of time. Each annotation point is marked on the respective signal trace by an icon, such as a large dot. The icons are useful in drawing the clinician's attention to the differences in LAT among the electrodes, but they tend to obscure the form and quality (morphology) of the signals around the annotation points. The icons can therefore make it difficult for the clinician to view and assess details of clinical significance in the electrogram morphology.

There is a need for a mode of electrophysiological signal processing and display in which annotations can be automatically identified and presented visually to the clinician without compromising the visibility of the morphology of the signal traces. The present disclosure addresses this need by displaying the signals such that the segments of the traces that are within a time window of a specified duration containing the respective annotation points are displayed with an increased brightness relative to parts of the traces outside the time window. The segments of the signals within the time window can thus be displayed without overlaying icons marking the annotation points on the traces. The brightened traces highlight the local activation times without cluttering the signal segments around the respective annotation points, thus drawing the clinician's attention to these segments while enhancing the clinician's appreciation of the morphology of the signals.

The examples described below relate particularly to processing and display of intracardiac electrograms. Alternatively, the principles of the present disclosure may be applied to other electrophysiological signals, and particularly signals generated by the heart, such as electrocardiogram signals acquired from the body surface

### SYSTEM DESCRIPTION

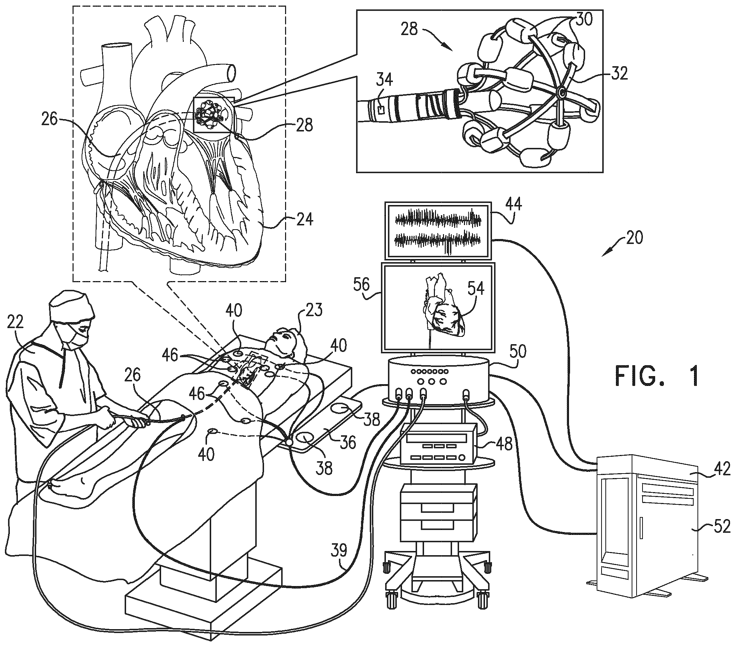

Fig. 1 shows an example catheter-based electrophysiology mapping and ablation system 20, in accordance with an example of the disclosure. System 20 may include multiple catheters, which are percutaneously inserted by a physician 22 through the vascular system of a patient 23 into a chamber or vascular structure of a heart 24. Typically, a delivery sheath (not shown) is inserted into the left or right atrium near a desired location in heart 24. Thereafter, one or more catheters 26 are inserted through the delivery sheath so as to arrive at the desired location in heart 24. The multiple catheters may include catheters dedicated for sensing intracardiac electrogram (IEGM) signals, catheters dedicated for ablating, and/or catheters used for both sensing and ablating. The distal part of catheter 26 in the pictured example comprises a basket assembly 28. Physician 22 may manipulate catheter 26 to place basket assembly 28 in contact with the heart wall for sensing a target site in heart 24 and/or for ablating tissue at the target site.

Catheter 26 is an exemplary catheter that includes multiple electrodes 30 distributed over a plurality of spines 32 in basket assembly 28 and configured to sense IEGM signals and/or ablate myocardial tissue. Catheter 26 additionally includes one or more position sensors 34 embedded in the distal part of the catheter for tracking the position and orientation of basket assembly 28, as described further hereinbelow. For example, position sensor 34 may comprise a magnetic position sensor including three magnetic coils for sensing three-dimensional (3D) position and orientation.

Magnetic position sensor 34 may be operated together with a location pad 36 including multiple magnetic coils 38 configured to generate magnetic fields in a predefined working volume containing heart 24. The position of basket assembly 28 of catheter 26 may be tracked based on magnetic fields generated by location pad 36 and sensed by magnetic position sensor 34 (which may include three orthogonal coils). Details of magnetic position sensing technology that may be applied for this purpose are described, for example, in U.S. Patents 5,5391,199; 5,443,489; 5,558,091; 6,172,499; 6,239,724; 6,332,089; 6,484,118; 6,618,612; 6,690,963; 6,788,967; and 6,892,091.

The multiple catheters may also include a reference catheter 39, which is percutaneously inserted by physician 22 through the vascular system of patient 23. Physician 23 brings an electrode at the distal end (not shown) of reference catheter 39 into contact with the coronary sinus of heart 24 of patient 23. Reference catheter 39 is typically left in place for the duration of the procedure and provides a reference timing signal to which the LAT measurements made by catheter 26 are referred.

System 20 optionally includes one or more electrode patches 40 in contact with the skin of patient 23 to establish location references for location pad 36, as well as for impedance-based tracking of electrodes 30. For impedance-based tracking, electrical current is directed to electrodes 30 and sensed at electrode patches 40 so that the location of each electrode 30 can be triangulated via electrode patches 40. Details of this sort of impedance-based location tracking technology are described in U.S. Patent 7,536,218; 7,756,576; 7,848,787; 7,869,865; and 8,456,182.

A recorder 42 records and displays electrograms 44 captured by body surface ECG electrodes 46 and intracardiac electrograms (IEGM) captured by electrodes 30 of catheter 26. Recorder 42 may include pacing capability for pacing the heart rhythm and/or may be electrically connected to a standalone pacer.

System 20 may include an ablation energy generator 48 for providing ablative energy to one or more of electrodes 30. Energy produced by ablation energy generator 48 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

A patient interface unit (PIU) 50 comprises an interface for electrical communication between catheters 26, other electrophysiological equipment, a power supply, and a workstation 52 for controlling operation of system 20. Electrophysiological equipment in system 20 may include for example, multiple catheters 26 (including catheters having other sorts of distal assemblies and electrode arrays, different from a basket catheter), location pad 36, body surface ECG electrodes 46, electrode patches 40, ablation energy generator 48, and recorder 42. Optionally, PIU 50 additionally includes processing capability for implementing real-time computations of the position of the catheters and for processing ECG signals.

Workstation 52 includes a memory and a processor, with appropriate operating software stored in the memory, and user interface capability. Workstation 52 may provide multiple functions, optionally including (1) modeling the endocardial anatomy in three-dimensions (3D) and rendering the model or an anatomical map 54 for display on a display screen 56; (2) displaying on display screen 56 activation sequences (or other data) compiled from recorded electrograms 44 in representative visual indicia or imagery superimposed on the rendered anatomical map 54; (3) displaying real-time location and orientation of one or more catheters within heart 24; and (4) displaying on display screen 56 sites of interest such as places where ablation energy has been applied. A commercial product embodying elements of system 20 is the CARTO^{®} 3 System, available from Biosense Webster, Inc. (31A Technology Drive, Irvine, CA 92618).

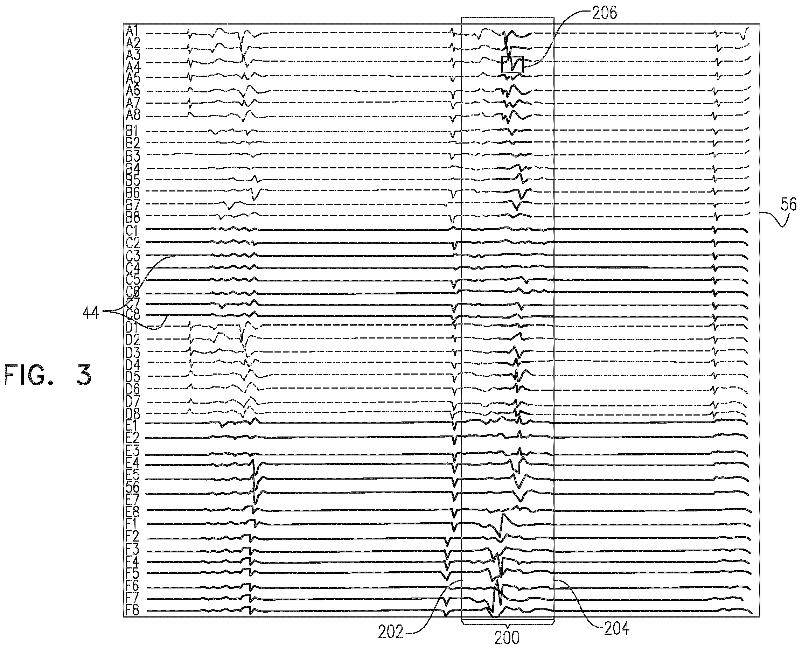

In examples of the present disclosure, workstation 52 processes the electrogram signals output by catheter 26 to find respective annotation points, corresponding to the local activation times, and to highlight the annotation points on display screen 56 by increasing the brightness of the traces within time windows containing the local activation times. This mode of display is described further hereinbelow with reference to Fig. 3.

### DISPLAY OF LOCAL ACTIVATION TIMES

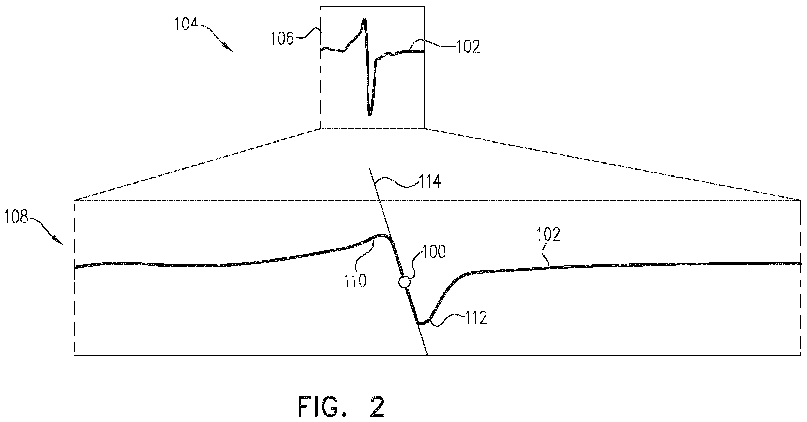

Fig. 2 is a plot that schematically illustrates the identification of an annotation point 100 in an example electrophysiological signal 102, in accordance with an example of the disclosure. A view 104 shows electrophysiological signal 102 within a window 106. For clarity, view 104 is stretched along the axis of time (horizontal axis) to a view 108. The processor within workstation 52 (Fig. 1) computes the location of annotation point 100 on signal 102 as the location between a positive peak 110 and a negative peak 112 where a slope 114 (time derivative) of the signal reaches an extreme negative value. This location corresponds to an inflection point of electrophysiological signal 102.

In more complex electrophysiological signals, the processor may use alternative algorithms for computing the location of their respective annotation points.

Fig. 3 is a schematic representation of a part of display screen 56 (Fig. 1), showing highlighted segments of recorded electrograms 44, in accordance with an example of the disclosure. Electrograms 44 are represented by corresponding traces on display screen 56 and are labeled along the left edge of the display by alphanumerical labels A1-A8, B1-B8, C1-C8, D1-D8, E1-E8, and F1-F8. A window of interest (WOI) 200 containing the local activation times of electrograms 44 is marked on electrograms 44 by two vertical lines 202 and 204. WOI 200 may be selected by an operator of the electrophysiology system (such as physician 22), or it may be selected automatically by the system.

The processor of workstation 52 has computed the locations of respective annotation points corresponding to the local activation times in electrograms A1-A8, B1-B8, and D1-D8. The processor has increased the brightness of the segments of the electrograms within a time window of a specified duration containing the identified local activation times relative to parts of the traces outside this time window. As an example, a bright segment of this sort is shown in a box 206 around electrogram A3, while the remainder of the trace within WOI 200 is dimmed. The parts of the traces outside WOI 200 may also be dimmed, or they may be displayed with full brightness. Some of the other electrograms show more complex signal forms, where the locations of the respective annotation points have been computed by the processor.

The temporal width of each bright segment around the annotation point is typically between about 10 and 100 ms. In the example shown in Fig. 3, the time window that is brightened in each trace has a duration of 15 ms. The LAT in each trace and the variation in LAT from trace to trace can be visualized readily, even without overlaying icons representing the local activation times on the traces.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method for electrophysiological measurement, comprising:
acquiring signals indicative of cardiac electrical activity from electrodes contacting tissues of a body of a living patient;
processing the signals to identify local activation times at one or more of the electrodes; and
displaying traces representing the signals as a function of time on a display screen, while increasing a brightness of segments of the traces within a time window of a specified duration containing the identified local activation times relative to parts of the traces outside the time window.

2. The method according to claim 1, wherein acquiring the signals comprises receiving intracardiac electrogram signals acquired within a heart of the living patient.

3. The method according to claim 1, wherein acquiring the signals comprises receiving electrocardiogram signals acquired from a body surface of the living patient.

4. The method according to claim 1, wherein the specified duration is between 10 ms and 100 ms.

5. The method according to claim 1, wherein displaying the traces comprises presenting the traces on the display screen without overlaying icons representing the local activation times on the traces.

6. Medical apparatus, comprising:
a display screen; and
a processor configured to:
acquire signals indicative of cardiac electrical activity from electrodes contacting tissues of a body of a living patient;
process the signals to identify local activation times at one or more of the electrodes; and
display traces representing the signals as a function of time on a display screen, while increasing a brightness of segments of the traces within a time window of a specified duration containing the identified local activation times relative to parts of the traces outside the time window.

7. The apparatus according to claim 6, wherein the signals comprise intracardiac electrogram signals acquired within a heart of the living patient.

8. The apparatus according to claim 6, wherein the signals comprise electrocardiogram signals acquired from a body surface of the living patient.

9. The apparatus according to claim 6, wherein the specified duration is between 10 ms and 100 ms.

10. The apparatus according to claim 6, wherein the processor is configured to present the traces on the display screen without overlaying icons representing the local activation times on the traces.

11. A computer-readable medium comprising instructions for electrophysiological measurement, the instructions when executed by a processor cause the processor to perform the steps of:
acquiring signals indicative of cardiac electrical activity from electrodes contacting tissues of a body of a living patient;
processing the signals to identify local activation times at one or more of the electrodes; and displaying traces representing the signals as a function of time on a display screen, while increasing a brightness of segments of the traces within a time window of a specified duration containing the identified local activation times relative to parts of the traces outside the time window.

12. The computer-readable medium according to claim 11, wherein the instructions for acquiring the signals comprise instructions for receiving intracardiac electrogram signals acquired within a heart of the living patient.

13. The computer-readable medium according to claim 11, wherein the instructions for acquiring the signals comprise instructions for receiving electrocardiogram signals acquired from a body surface of the living patient.

14. The computer-readable medium according to claim 11, wherein the instructions for the specified duration set a range between 10 ms and 100 ms.

15. The computer-readable medium according to claim 11, wherein the instructions for displaying the traces comprise instructions for presenting the traces on the display screen without overlaying icons representing the local activation times on the traces.
